# EUROPEAN PATENT APPLICATION

(11) **EP 1 502 960 A1**
(43) Date of publication of application: **02.02.2005**
(21) Application number: 03723207.1
(22) Date of filing: 25.04.2003
(51) Int. Cl.: C12Q 1/68

(54) **METHOD OF DETECTING RHEUMATOID ARTHRITIS BY DETECTING OVEREXPRESSION OF WNT**

(30) Priority: 02.05.2002 JP 2002130883
(71) Applicant: Japan Science and Technology Agency, Kawaguchi-shi, Saitama 332-0012 (JP)
(72) Inventor: IMAI, Kazushi, Shinjuku-ku, Tokyo 162-0067 (JP)
(74) Representative: Duckworth, Timothy John
(86) International application number: PCT/JP2003/005358
(87) International publication number: WO 2003/093508

(57) **Abstract**

A convenient and highly accurate pre-examination of detecting rheumatoid arthritis wherein at least the upregulation of expression of WNT at least the upregulation of expression of WNT10B and at least the regulated expression of FRP1 in joint synovial liquid, joint synovial tissue or peripheral blood are detected in parallel.

SEQUENCE LISTING

<110>  Japan Science and Technology Agency

<120>  METHOD OF DETECTING RHEUMATOID ARTHRITIS BY DETECTING
       OVEREXPRESSION OF WNT

<130>  N.93241

<160>  44

<170>  PatentIn version 3.2

<210>  1
<211>  20
<212>  DNA
<213>  Artificial sequence

<220>
<223>  primer

<400>  1
tcctgctcag aaggttccat                                                20


<210>  2
<211>  20
<212>  DNA
<213>  Artificial sequence

<220>
<223>  primer

<400>  2
gctgtacgtg cagaagttgg                                                20


<210>  3
<211>  20
<212>  DNA
<213>  Artificial sequence

<220>
<223>  primer

<400>  3
ctgtatcagg gaccgagagg                                                20


<210>  4
<211>  20
<212>  DNA
<213>  Artificial sequence

<220>
<223>  primer

<400>  4
caaagagaac tcgccaggag                                                20

<210>  5
<211>  20
<212>  DNA
<213>  Artificial sequence

<220>
<223>  primer

<400>  5
actgagtgtg tgcagctgtg                                                    20


<210>  6
<211>  20
<212>  DNA
<213>  Artificial sequence

<220>
<223>  primer

<400>  6
tgatgtcttg ctgcagacac                                                    20


<210>  7
<211>  20
<212>  DNA
<213>  Artificial sequence

<220>
<223>  primer

<400>  7
acttcggcgt gttagtctcc                                                    20


<210>  8
<211>  20
<212>  DNA
<213>  Artificial sequence

<220>
<223>  primer

<400>  8
atttttcctt ccgcttctcc                                                    20


<210>  9
<211>  20
<212>  DNA
<213>  Artificial sequence

<220>
<223>  primer

<400>  9
ttgaggagtg ccactaccag                                                    20

<210> 10
<211> 20
<212> DNA
<213> Artificial sequence

<220>
<223> primer

<400> 10
ttgaactgtg cgttgcgtgg 20


<210> 11
<211> 20
<212> DNA
<213> Artificial sequence

<220>
<223> primer

<400> 11
cagttcaaga ccgtgcagac 20


<210> 12
<211> 20
<212> DNA
<213> Artificial sequence

<220>
<223> primer

<400> 12
tggaacctac ccatcccata 20


<210> 13
<211> 20
<212> DNA
<213> Artificial sequence

<220>
<223> primer

<400> 13
gtgctgcttc gtcaggtgta 20


<210> 14
<211> 20
<212> DNA
<213> Artificial sequence

<220>
<223> primer

<400> 14
cgaggttgaa gctgagttcc 20

<210>  15
<211>  20
<212>  DNA
<213>  Artificial sequence

<220>
<223>  primer

<400>  15
caactgcaca acaacgaggc                                                    20


<210>  16
<211>  20
<212>  DNA
<213>  Artificial sequence

<220>
<223>  primer

<400>  16
gtactacgca gcaccagtgg                                                    20


<210>  17
<211>  20
<212>  DNA
<213>  Artificial sequence

<220>
<223>  primer

<400>  17
gagaagcaag gccagtacca                                                    20


<210>  18
<211>  20
<212>  DNA
<213>  Artificial sequence

<220>
<223>  primer

<400>  18
acagcacatg aggtcacagc                                                    20


<210>  19
<211>  20
<212>  DNA
<213>  Artificial sequence

<220>
<223>  primer

<400>  19
acatgctatc agctctgctg                                                    20

<210>  20
<211>  20
<212>  DNA
<213>  Artificial sequence

<220>
<223>  primer

<400>  20
aaagatcagt tccgcctctg                                                    20


<210>  21
<211>  20
<212>  DNA
<213>  Artificial sequence

<220>
<223>  primer

<400>  21
gaaagtggca agctttggag                                                    20


<210>  22
<211>  20
<212>  DNA
<213>  Artificial sequence

<220>
<223>  primer

<400>  22
gaaagtggca agctttggag                                                    20


<210>  23
<211>  20
<212>  DNA
<213>  Artificial sequence

<220>
<223>  primer

<400>  23
aatgaggctt cacaacaacc                                                    20


<210>  24
<211>  20
<212>  DNA
<213>  Artificial sequence

<220>
<223>  primer

<400>  24
tcatgtggtc caatctcctc                                                    20

<210> 25
<211> 20
<212> DNA
<213> Artificial sequence

<220>
<223> primer

<400> 25
cttcattgat acccacaacc 20


<210> 26
<211> 20
<212> DNA
<213> Artificial sequence

<220>
<223> primer

<400> 26
attgttgggg agaaggctac 20


<210> 27
<211> 20
<212> DNA
<213> Artificial sequence

<220>
<223> primer

<400> 27
tgacctcaag acccgatacc 20


<210> 28
<211> 20
<212> DNA
<213> Artificial sequence

<220>
<223> primer

<400> 28
caagtgaagg caaagcacaa 20


<210> 29
<211> 20
<212> DNA
<213> Artificial sequence

<220>
<223> primer

<400> 29
aagatggtgc caacttcacc 20

<210>  30
<211>  20
<212>  DNA
<213>  Artificial sequence

<220>
<223>  primer

<400>  30
taaggaacca gccaggacac                                                    20


<210>  31
<211>  20
<212>  DNA
<213>  Artificial sequence

<220>
<223>  primer

<400>  31
gtgacaccac cttgcagaac                                                    20


<210>  32
<211>  20
<212>  DNA
<213>  Artificial sequence

<220>
<223>  primer

<400>  32
accctctgat gtacggttgc                                                    20


<210>  33
<211>  20
<212>  DNA
<213>  Artificial sequence

<220>
<223>  primer

<400>  33
cttgttcttg cagcattccc                                                    20


<210>  34
<211>  20
<212>  DNA
<213>  Artificial sequence

<220>
<223>  primer

<400>  34
agagaaggca atgcctctcc                                                    20

<210>  35
<211>  20
<212>  DNA
<213>  Artificial sequence

<220>
<223>  primer

<400>  35
aaagacagct tgcagtgcac                                                    20


<210>  36
<211>  20
<212>  DNA
<213>  Artificial sequence

<220>
<223>  primer

<400>  36
tgttatgaca acctcagtgg                                                    20


<210>  37
<211>  20
<212>  DNA
<213>  Artificial sequence

<220>
<223>  primer

<400>  37
cattgacttc cagcacgagc                                                    20


<210>  38
<211>  20
<212>  DNA
<213>  Artificial sequence

<220>
<223>  primer

<400>  38
acgaagcttc atatcccagc                                                    20


<210>  39
<211>  20
<212>  DNA
<213>  Artificial sequence

<220>
<223>  primer

<400>  39
agaggagtgg ctgcaatgag                                                    20

<210>  40
<211>  20
<212>  DNA
<213>  Artificial sequence

<220>
<223>  primer

<400>  40
tggccttaca taggctgtcc                                                    20


<210>  41
<211>  20
<212>  DNA
<213>  Artificial sequence

<220>
<223>  primer

<400>  41
aagtggatgg acagctgctg                                                    20


<210>  42
<211>  20
<212>  DNA
<213>  Artificial sequence

<220>
<223>  primer

<400>  42
tactttctga gaccctgagg                                                    20


<210>  43
<211>  20
<212>  DNA
<213>  Artificial sequence

<220>
<223>  primer

<400>  43
gtcagtggtg gacctgacct                                                    20


<210>  44
<211>  20
<212>  DNA
<213>  Artificial sequence

<220>
<223>  primer

<400>  44
aggggagctt cagtgtggtg                                                    20

## Description

### FIELD OF THE INVENTION

The present invention relates to the method for detection of rheumatoid arthritis by monitoring the upregulation of expression of *WNT*, especially *WNT* 10B, in joint synovial fluid or in peripheral blood by a reverse transcription (RT) PCR analysis. In addition, it includes the method for detection of secreted *frizzled-related protein* (*FRP*) in parallel with that of *WNT*.

### BACKGROUND OF THE ART

For the diagnosis of rheumatoid arthritis (RA), the criteria which was advocated by the American College of Rheumatology is adapted worldwide. The criteria consists of, 1) stiffness which continues more than one hour in the morning, 2) swelling in more than three joints, 3) swelling and deformity in joints of the hand, 4) symmetrical swelling of the joint, 5) aberration of the hand joints verified by radiography, 6) subcutaneous nodules, and 7) positive reaction of rheumatoid factor by blood examination. To diagnose patients as RA, it is required to ascertain more than 4 criteria, and the criteria 1) - 4) should continue over 6 weeks. Since disease onset is insidious in most cases and the diagnosis criteria is largely depending on clinical findings, several months or years can elapse before a firm diagnosis can be ascertained. The delay of the diagnosis reduces the effectiveness of conservative therapies, and urge patients to burden with the mental, physical and economical demands.

As a biochemical examination, measuring the serum levels of rheumatoid factor are widely utilized to predict the onset of RA. However, it is a controversial issue that the rheumatoid factor can reflect the onset because of high frequency of false-positive and false-negative cases.

Therefore, there is an urgent need to develop a novel strategy that makes possible to diagnose patients suffered from RA in the early stage with high specificity (http://www.rheuma-net.or.jp/).

The *WNT* family was cloned as an oncogenic gene family and consists of 19 paralogues in the human genome. It stimulates an expression of c-Myc and Cyclin D 1 and enhances proliferation of tumor cells and endothelial cells (Wright, M., Aikawa, M. Szto, W. and Papkoff, J. Identification of a WNT-responsive signal transduction pathway in primary endothelial cells. Biochem. Biophys. Res. Commun. 263:384-388, 1999). Recently, it is reported that RA synovium increase the expression of *WNT 1* and *WNT5A*, and the production of inflammatory cytokines including Interleukin 6, 8, and 15 (refer to Sen. M., Lauterbach, K., EI-Gabawy, H., Firestein, G.S., Corr, M. and Carson, D. A. Expression and function of wingless and frizzled homologs in rheumatoid arthritis. Proc. Natl. ACAD. Sci. USA. 97: 2791-2796, 2000). In contrast, the synovial tissue from osteoarthritic (OA) patients did not express *WNT*1 and *WNT5A*.

Further, in Japanese Patent Laid-Open Publication 11-113580, specifically, in lines 1-3 of the fourth column, the art regarding diagnostic assay for diseases relating WNT-11 activity or level is referred, and from □0033□ to □0034□said diagnostic assay is explained. However, there is no refer explaining that the RA specific diagnosis is possible by detecting the RA specific expression of *WNT*, especially *WNT*10B. Further, the expression of WNT11 at RA joint synovium, and the diagnostic assay by WNT11 detection is not included in the RA specific diagnosis method of the present invention.

The subject of the present invention is the providing of RA specific diagnosis method which detects the upregulation of *WNT* expression in the joint synovial tissue and fluids and peripheral blood, and enable us to diagnose RA in the early stage and to start the preventive therapeutics.

WNT is expressed in various types of developing organs and plays a pivotal role in the organogenesis and morphogenesis of embryonic stage (Moon, R. A. Brown, J. D. and Torres, M. WNTs modulate cell fate and behavior during vertebrate development. Trends Genet. 13: 157-162, 1997.). However, since the expression ceases after the completion of organogenesis, it is predicted that WNT does not have a predominant role in maintaining the integrity of adult organs. Therefore, aberrant activation of the WNT expression may contribute to cause or stimulate the pathological changes observed in RA synovium.

The inventor of the present invention, has clarified and compared the profiling of expression pattern of *WNTs* and *FRPs*, specific inhibitors of the WNT family, between patients with RA in which the pathological changes of synovium is associated and with OA which is not related to the synovial abnormalities. *WNT10B* was specifically expressed in RA synovium but other *WNT* members were less frequently or negligibly expressed in RA and OA synovium. In addition, WNT10B specifically localized to synovial surface cells and endothelial cells in RA tissues.

On the contrary, *FRPs* were specifically expressed OA synovium, especially, *FRP1* expression was identified in all of the OA tissues, and localized in synovial surface cells and endothelial cells, which were identical to that of RA tissues. Above data predicts that the expression of FRPs may prevent the action of WNT and the subsequent pathological activation.

Since the expression of *WNT10B* and *FRP1* discriminates the nature of RA and OA synovium, a novel RA-specific diagnosis method could be established by detecting the presence of *WNT* and *FRP,* in particular *WNT10B* and *FRP1*, in joint synovial fluid or peripheral blood, and the subject of the present invention can be dissolved.

### DISCLOSURE OF THE INVENTION

The present invention is (1) a method to detect rheumatoid arthritis by detecting at least the upregulation of expression of *WNT*10B in joint synovial fluid, in joint synovial tissue or in peripheral blood. Desirably, the present invention is (2) the method to detect rheumatoid arthritis of (1), wherein at least the upregulation of expression of *WNT10B* is detected by RT-PCR analysis. More desirably, the present invention is (3) the method to detect rheumatoid arthritis of (1) or (2), wherein at least inhabitation of expression of *FRP* is detected in parallel. Furthermore desirably, the present invention is (4) the method to detect rheumatoid arthritis of (3), wherein at least an inhabitation of expression *of FRP* is detected in parallel.

### BRIEF ILLUSTRATION OF THE DRAWING

Fig.1 shows the expression of WNT gene by RT-PCR analysis. Lanes 1-5 indicate the expression of *WNT*3, *WNT5A*, *WNT10B* and *WNT14* in RA synovial tissue, and lanes 6-9 indicate expression of *WNT*3, *WNT5A*, *WNT10B* and *WNT14* in OA synovial tissue.

Fig.2 shows the expression of *FRP* gene by RT-PCR analysis. Lanes 1-5 indicate the expression of *FRP1*, *FRP2*, *FRP3* and *FRP4* and *FRP5* in RA synovial tissue, and lanes 6-9 indicate expression of *FRP1*, *FRP2*, *FRP3, FRP4* and *FRP5* in OA synovial tissue.

### DESCRIPTION OF THE PREFERRED EMBODYMENT

The present invention will be illustrated in more in detail. 1. Profiling the expression pattern of *WNTs* and their specific inhibitors, *FRPs*, which were specifically detected in RA and OA synovium, respectively.
(1) Total RNA was isolated from patients suffered from RA (five cases) or OA (four cases) undergoing total joint replacement surgery according to the Chomczynski and Sacchi method. To prevent contamination of genomic DNA, RNA samples were treated by RNase-free DNaseI at 37°C for 30 minutes.
(2) 1^{st} strand cDNA (50 µl) was synthesized from 5µg of total RNA using oligo d(T) primer (Gibco BRL, Giathusberg, Germany) and reverse transcriptase (SuperScriptII, Gibco BRL). After incubation of 1µl of cDNA at 94°C for 5 minutes, PCR reaction was carried out using gene-specific primer sets, novel base series indicated in Table 1, and Taq DNA polymerase (Gibco BRL). Denaturing was carried out on 50µl of each reaction mixtures at 94°C for 30 seconds, at proper annealing temperature indicated in Table 1 for 30 seconds, and extension at 72°C for one minute. These procedures were set as the one cycle, and additional 29 cycles were repeated. The reaction mixture was analyzed by gel electrophoresis on 2% agarose gels and the presence of gene-specific amplification was confirmed.

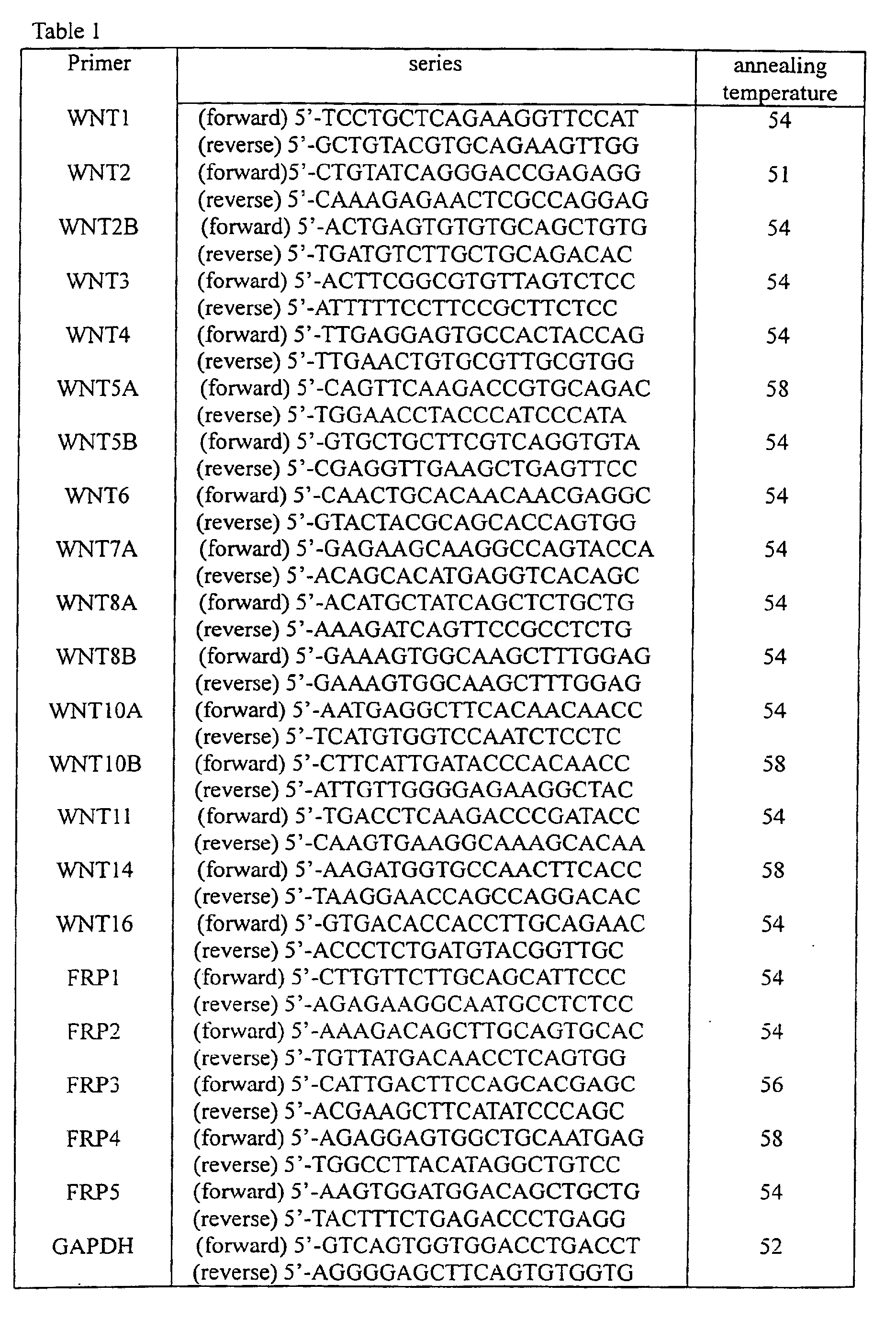

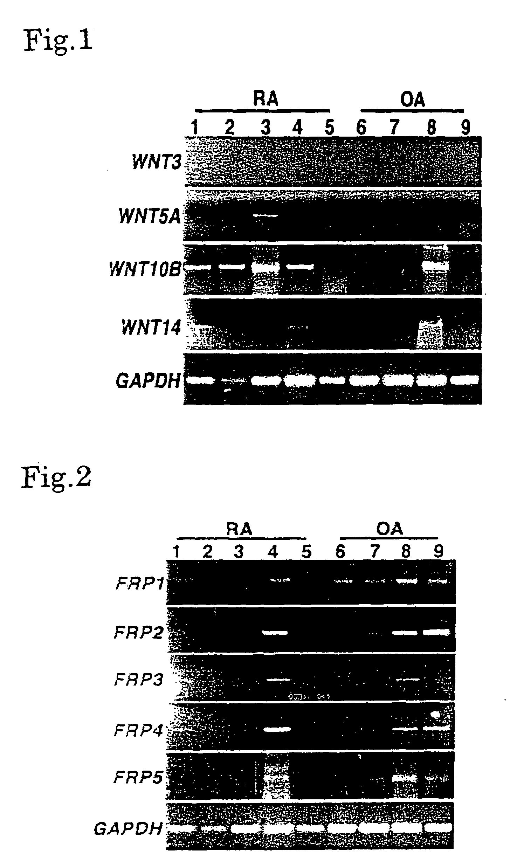

*WNT*10B was detected in four of five RA synovium but limited to one of four OA cases by RT-PCR. Little or no expression of other *WNT* members was detected. Representative results by RT-PCR were shown in Fig.1. *FRP*1 was detected in all of four OA cases analyzed, *FRP*2 and *FRP4* in 2/4 cases, *FRP*3 and *FRP*5 were observed in 1/4 case. In RA samples, each FRP gene was expressed in 1/5 case or not expressed. Expression of *FRPs* was represented in Fig.2. Table 2 summarized the results of RT-PCR: +; presence of expression, -; absence of expression, ND; not determined.

**Table 2**

| | RA | | | | | OA | | | |
|---|---|---|---|---|---|---|---|---|---|
| | 1 | 2 | 3 | 4 | 5 | 6 | 7 | 8 | 9 |
| WNT1 | - | - | - | - | - | - | ND | - | - |
| WNT2 | - | - | - | - | - | - | ND | - | - |
| WNT2B | - | - | - | - | - | - | ND | - | - |
| WNT3 | - | - | - | - | - | - | - | - | - |
| WNT4 | - | - | - | - | - | - | ND | - | - |
| WNT5A | - | - | + | - | - | - | - | - | - |
| WNT5B | - | - | - | + | - | - | ND | + | - |
| WNT6 | - | - | - | - | - | - | ND | - | - |
| WNT7A | - | - | - | - | - | - | ND | - | - |
| WNT8A | - | - | - | - | - | - | ND | - | - |
| WNT8B | - | - | - | - | - | - | ND | - | - |
| WNT10A | - | - | - | - | - | - | ND | - | - |
| WNT10B | + | + | + | + | - | - | - | + | - |
| WNT11 | - | - | - | - | - | - | ND | + | - |
| WNT14 | - | - | - | + | - | - | - | + | - |
| FRP1 | - | - | - | + | - | + | + | + | + |
| FRP2 | - | - | - | + | - | - | - | + | + |
| FRP3 | - | - | - | - | - | - | - | + | - |
| FRP4 | - | - | - | + | - | - | - | + | + |
| FRP5 | - | - | - | - | - | - | - | + | - |

Formalin- or paraformaldehyde-fixed and paraffin-embedded tissue sections of human RA or OA synovium were deparaffinized, rehydrated in ethanol series, and treated in a microwave oven (500 W, 4 minutes, 3 times) in 0.01 M sodium citrate buffer (pH6.0). After the treatment, the tissue sections was incubated with normal goat serum, normal donkey serum, normal rabbit serum or 1% bovine serum albumin at room temperature for 30 minutes, and with goat anti-Wnt10b antibody (1 µg/ml, Santa Cruz Biotechnology, Santa Cruz, CA, U.S.A.), goat anti-Frp1 antibody (2 µg/ml, Santa Cruz Biotechnology, Santa Cruz, CA, U.S.A.), or rabbit anti-von Willbrand Factor (vWF) antibody (200-fold dilution, DAKO, Carpinteria, CA, U.S.A.). And then, incubated with Alexa Fluor 546 anti-rabbit IgG, Alexa Flour 546 anti-goat IgG (Molecular Probes, Eugene, OR, U.S.A.), or FITC-labeled anti-goat IgG (Vector, Burlingame, CA, U.S.A.) at room temperature for 90 minutes, and tissue localization of each antigen was examined by a fluorescene microscope.

In RA synovium, *WNT* 10B localized in synovial surface cells and endothelial cells. In OA synovium, the reaction was not observed. On the contrary, FRP 1 localized in synovial surface cells and endothelial cells in OA tissues, but not in RA tissues.

Localization of WNT10B and FRP1 in endothelial cells was confirmed by a double immunostaining technique using anti-vWF antibody, which specifically recognizes endothelial cells.

From the immunohistostaining micrograms obtained by said staining technique, WNT10B-positive cells in RA synovium and FRP1-positive cell in OA synovium, which confirmed the specific expression pattern in RA and OA synovial tissues, respectively.

### POSSIBILITY FOR THE INDUSTRIAL APPLICABILITY

By the present invention, the profiling of expression pattern of WNTs and FRPs in RA and OA synovial tissues was clarified. That is, by RT-PCR analysis using gene-specific primer sets, especially, by the positive and/or negative of amplification of the genes by RT- PCR analysis for *WNT*10B or combinatorial detection of *WNT*10B and *FRP*1, the early RA specific diagnosis without false-positive reaction is accomplished. Accordingly, easy and reliable RA-specific diagnosis can be accomplished, and can provide an excellent technique which has high industrial applicability.

## Claims

1. A method to detect rheumatoid arthritis by detecting at least the upregulation of expression of *WNT*10B in joint synovial fluid, in joint synovial tissue or in peripheral blood.

2. The method to detect rheumatoid arthritis of claim 1, wherein at least the upregulation of expression of *WNT10B* is detected by RT-PCR analysis.

3. The method to detect rheumatoid arthritis of claim 1, wherein at least inhabitation of expression of FRP is detected in parallel.

4. The method to detect rheumatoid arthritis of claim 2, wherein at least an inhabitation of expression of FRP is detected in parallel.
